# EUROPEAN PATENT APPLICATION

(11) **EP 1 369 132 A1**
(43) Date of publication of application: **10.12.2003**
(21) Application number: 02024545.2
(22) Date of filing: 31.10.2002
(51) Int. Cl.: A61K 47/48, A61K 31/282

(54) **Oxaliplatin containing liposome preparation**

(30) Priority: 03.06.2002 JP 2002161296
(71) Applicant: Mebiopharm Co., Ltd., Tokyo (JP)
(72) Inventor: Eriguchi, Masazumi, Shinagawa-ku, Tokyo (JP); Yanagie, Hironobu, Minato-ku, Tokyo (JP); Maruyama, Kazuo, Sagamihara-shi, Kanagawa (JP); Fujisawa, Tadashi, Minato-ku, Tokyo (JP)
(74) Representative: VOSSIUS & PARTNER

(57) **Abstract**

The present invention provides a liposome preparation containing oxaliplatin and derivatized with a hydrophilic polymer, as well as a pharmaceutical composition for treatment of tumor comprising the liposome preparation. The liposome preparation according to the present invention is characterized in that it is derivatized with a ligand. The ligand is preferably transferrin. In accordance with the invention, the uptake of a pharmaceutical agent in the liposome into tumor cells can be enhanced through transferrin receptors expressed on the surface of the tumor cells.

## Description

### FIELD OF THE INVENTION

The present invention relates to a liposome preparation for use as an anti-tumor agent.

### BACKGROUND OF THE INVENTION

Cisplatin has been widely used as an anti-tumor agent for the treatment of various cancers including testis tumor, bladder tumor, renal pelvis and ureter tumor, prostate cancer, ovarian cancer, head and neck cancer, non-small cell lung cancer, esophageal cancer, cervical cancer, neuroblastoma and gastric cancer. However, cisplatin has disadvantages in that it is highly toxic and is usually associated with adverse side effects such as renal disorders including acute renal failure, inhibition of the bone marrow function, nausea, vomiting and anorexia. For the purpose of overcoming these disadvantages, cisplatin derivatives such as carboplatin and oxaliplatin have been developed. Oxaliplatin exerts therapeutic activities similar to those of cisplatin and has relatively low nephrotoxicity and emetogenicity.

For the purpose of reducing the toxicity of an anti-tumor agent to normal cells, the anti-tumor agent is often encapsulated in liposomes for administration. However, this technique needs further improvement in entrapment of an anti-tumor agent in liposomes, stability of the resultant liposome preparation and efficient delivery of the liposome preparation to target cells.

Accordingly, technologies for improving the storage stability of a liposome preparation containing an anti-tumor agent and the delivery efficiency of the therapeutic agent in the liposome preparation to target tumor cells are needed in the art.

The object of the present invention is to provide an oxaliplatin-containing liposome preparation having high stability and efficiency.

### SUMMARY OF THE INVENTION

The present invention provides a liposome preparation containing oxaliplatin, which is derivatized with a hydrophilic polymer and a ligand.

The ligand is preferably selected from the group consisting of transferrin, folic acid, hyaluronic acid, a sugar chain such as galactose and mannose, a monoclonal antibody and a Fab' fragment of a monoclonal antibody.-Particularly preferably, the ligand is transferrin.

The hydrophilic polymer is preferably selected from the group consisting of polyethylene glycol, polymethylethylene glycol, polyhydroxypropylene glycol, polypropylene glycol, polymethylpropylene glycol and polyhydroxypropylene oxide. More preferably, the hydrophilic polymer is polyethylene glycol.

The present invention also provides a pharmaceutical composition for the treatment of tumors, comprising a liposome preparation containing oxaliplatin and derivatized with a hydrophilic polymer and a ligand, and a pharmaceutically acceptable carrier.

### BRIEF DESCRIPTION OF THE DRAWINGS

Fig. 1 is a schematic representation showing the production process for a transferrin-conjugated liposome according to the present invention.
Fig. 2 is a graph showing the cytotoxicity of oxaliplatin.
Fig. 3 is a table showing the characteristic properties of a unmodified liposome, a PEG liposome and a Tf-PEG liposome.
Fig. 4 is a graph showing the number of transferrin receptors present on the cell surface in each of normal leukocytes and cells of various types of tumor-derived cell lines.
Fig. 5 is a table showing the production of bloody ascites and tumor nudules in mice administered with each of a unmodified liposome, a PEG liposome and a Tf-PEG liposome.

### DETAILED DESCRIPTION OF THE PREFERRED EMBODIMENTS

A liposome is a spherical lipid bilayer having an inner-aqueous core. During the formation of a liposome, molecules in an aqueous solution are entrapped in the inner aqueous core. The content of the liposome can be protected against the external micro-environment and transported efficiently into the cytoplasm upon the fusion of the liposome to the cell membrane.

In one aspect, the present invention provides a liposome preparation containing oxaliplatin, which is derivatized with a hydrophilic polymer and a ligand. Oxaliplatin, a platinum (II) cis-oxalato complex of trans-1-1,2-diaminocyclohexane, is a platinum complex compound represented by the following formula:

### <FORMULA>

Oxaliplatin is useful as an anti-tumor agent, since it has therapeutic activities similar to those of cisplatin and relatively low nephrotoxicity and emetogenicity. The production process for oxaliplatin is well known in the art (see, for example, Japanese Patent Public Disclosure No. 9-40685). In the liposome preparation according to the present invention, it is preferred that oxaliplatin be entrapped in liposomes in the form of an aqueous solution at a concentration of 1 to 20 mg/ml.

The liposome preparation can be produced by dissolving a phospholipid in a suitable organic solvent, dispersing the resultant solution in an aqueous solution containing a therapeutic agent, and then performing ultrasonication or reverse phase evaporation of the resultant dispersion. The phospholipid used in accordance with the invention includes, for example, phosphatidylcholine, phosphatidylethanolamine, phosphatidylinositol, sphingomyelin or phosphatidic acid. For the purpose of stabilizing the lipid membrane, it is preferred to add an additional lipid component, such as cholesterol.

In addition, in order to prevent the uptake of the liposomes into the cellular endothelial systems and enhance the uptake of the liposomes into the tumor tissues, the outer surface of the liposomes may be modified with a hydrophilic polymer. The modification of the liposomes with a hydrophilic polymer is known to enable to prolong the half-life of the liposomes in the blood. Examples of the hydrophilic polymer include polyethylene glycol, polymethylethylene glycol, polyhydroxypropylene glycol, polypropylene glycol, polymethylpropylene glycol and polyhydroxypropylene oxide. A particularly preferred hydrophilic polymer is polyethylene glycol.

The liposome preparation of the present invention is characterized in that it is further derivatized with a ligand. A ligand refers to a substance that can attach to a receptor or surface antigen on the cell surface. Preferably, the ligand is selected from the group consisting of transferrin, folic acid, hyaluronic acid, a sugar chain such as galactose and mannose, a monoclonal antibody and a Fab' fragment of a monoclonal antibody.

In the particularly preferred embodiments of the present invention, the ligand is transferrin. Transferrin is an iron-binding protein found in vivo. Upon attaching to a transferrin receptor on the surface of cells so as to be taken up into the cells, transferrin acts to supply iron to the cells. The transferrin receptor is generally expressed in tumor tissues in a larger amount, regardless of the types of the tumor, compared with normal tissues. Therefore, by binding a therapeutic agent to transferrin, uptake of the therapeutic agent into tumor cells may be enhanced through the transferrin receptor.

Preferably, the liposome preparation of the present invention contains 1 to 20 µg/mg lipid of oxaliplatin and 100 to 300 µg/mg lipid of a ligand.

The liposome preparation of the present invention can be produced by reverse phase evaporation (REV) method (see US Patent No. 4,235,871). In order to stably retain the hydrophilic polymer within the lipid bilayer, it is preferred to previously prepare a phospholipid derivative of the hydrophilic polymer, and then using the phospholipid derivative together with a phospholipid and a lipid to prepare the liposome. The phospholipid derivative of the hydrophilic polymer may be prepared in such a manner as described in, for example, US Patent No. 5,013,556. Briefly, a hydrophilic polymer such as polyethylene glycol is treated with cyanuric acid in a basic organic solvent to activate one terminus of the hydrophilic polymer, and the resultant product is then reacted with a phospholipid such as phosphatidylethanol, thereby obtaining a phospholipid derivative of the hydrophilic polymer. It is preferred that the other terminus of the hydrophilic polymer have a functional group, such as a carboxyl or maleimide group, to which the ligand is to be attached.

A phospholipid (e.g., distearoyl phosphatidylcholine), a lipid (e.g., cholesterol) and a phospholipid derivative of the hydrophilic polymer. (e.g., polyethylene glycol-phosphatidylethanolamine) are mixed together and then dissolved in a suitable organic solvent. The phospholipid and the lipid may be mixed at a ratio of 3:1 to 1:3, preferably 2:1 to 1:1. The phospholipid derivative of the hydrophilic polymer may be mixed at 1 to 10%, preferably about 5%, of the phospholipid. The resultant solution is mixed with a solution of oxaliplatin in an aqueous buffer. The concentration of oxaliplatin in the aqueous solution may be from 1 to 20 mg/ml, preferably from 5 to 10 mg/ml. The solvent mixture is sonicated and then evaporated to remove the solvent. The liposomes thus prepared is size-fractionated to afford oxaliplatin-containing liposomes having about 0.2 µm in diameter.

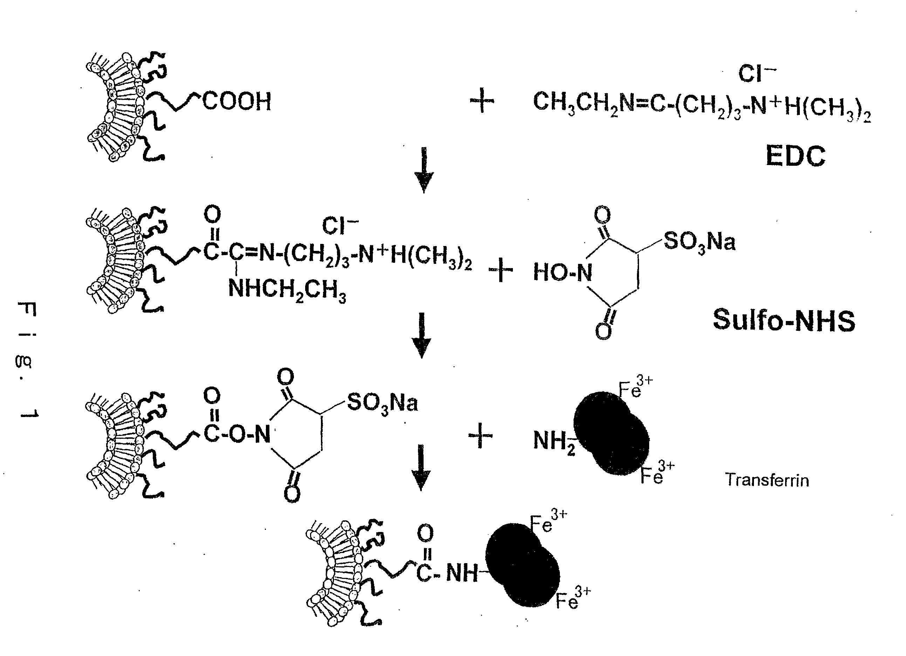

Subsequently, the ligand is attached to the outer surface of the liposomes. For example, in the case where transferrin is used as the ligand, the transferrin may be commercially available in the form of a purified protein. For the attachment of transferrin to the outer surface of the liposomes, it is preferred to previously introduce an additional functional group to the phospholipid derivative of the hydrophilic polymer. In a non-limiting example, a phospholipid derivative of a hydrophilic polymer which has a carboxyl or maleimide group introduced at its one terminus is added to a phospholipid to form liposomes having carboxyl or maleimide groups on the outer surface. in the case where the terminus has a carboxyl group, 1-ethyl-3-(3-dimethylamino-propyl)carbodiimido hydrochloride and N-hydroxysulfosuccineimide are bound to the liposomes. The resultant linker-attached liposomes are reacted with transferrin to obtain apo-form of transferrin-bound liposomes in which transferrin is bound to the outer surface. The resultant liposomes are treated with iron citrate/sodium citrate to obtain holo-form of transferrin-bound liposomes (Fig. 1). In the case where the terminus has a maleimide group, the linker-bound liposomes are reacted with transferrin having a SH group previously introduced therein, followed by the addition of iron in the same manner as described above to obtain holo-form of transferrin-bound liposomes.

In another aspect, the present invention provides a pharmaceutical composition for the treatment of a tumor, comprising the liposome preparation of the present invention and a pharmaceutically acceptable carrier. The carrier includes, for example, sterile water, a buffer solution and saline. The pharmaceutical composition may further comprise various salts, sugars, proteins, starch, gelatin, plant oils, polyethylene glycol. The composition of the present invention can be administered parenterally via bolus injection or continuous injection. The dosage may vary depending on the route of administration, the severity of the condition, the age and condition of the patient to be treated, and the degree of side effects, but is generally within the range from 10 to 100 mg/m²/day.

The disclosure of all patens and documents cited herein are entirely incorporated herein as reference. The present application claims priority based on Japanese Patent Application No. 2002-161296, the disclosure of which is entirely incorporated herein as reference.

### EXAMPLES

The following examples further illustrate the present invention. The examples below are not limiting and are merely representative of various aspects and features of the present invention.

### EXAMPLE 1: Cytotoxicity test for oxaliplatin

An oxaliplatin (I-OHP) solution was prepared by dissolving oxaliplatin in a 9% sucrose solution at a concentration of 8 mg/ml. The cell viability was determined using a commercially available cytotoxicity assay kit. AsPC-1 cells cultured in RPMI 11640 medium supplemented with 10% FCS were treated in each of different concentrations of I-OHP solutions at 37°C in 5% CO₂ for 48 hours. The medium was removed, and a substrate was added to the cells and incubated in 5% CO₂ for 2 hours. Color-developed was measured at the absorbance of 450 nm (reference wavelength: 620 nm).

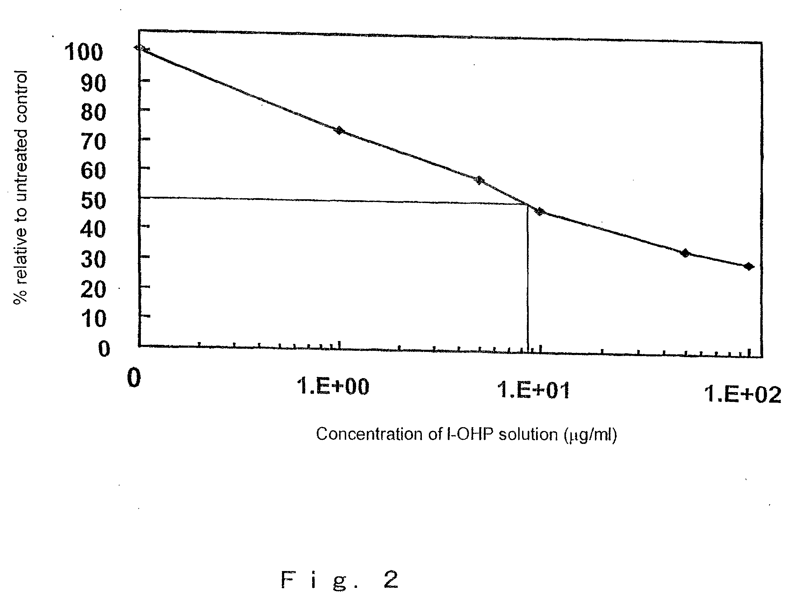

The results are shown in Fig. 2. The cytotoxicity of I-OHP was found to be LD50 > 8 µg/ml.

### EXAMPLE 2: Preparation of oxaliplatin-containing liposome

The composition of the liposome was as follows:
Distearoyl phosphatidylcholine (DSPC)
Cholesterol (CH)
N-(Carbamoylmethoxypolyethylene glycol 2000)-distearoyl phosphatidylethanloamine (DSPE-PEG-OMe)
Carboxyl polyethylene glycol 3000)-distearoyl phosphatidylethanolamine (DSPE-PEG-COOH)

DSPC:CH:DSPE-PEG-OMe:DSPE-PEG-COOH = 2:1:0.19:0.01 (m/m).

As the aqueous phase, a I-OHP solution (8 mg/ml, in a 9% sucrose solution) was used.

A mixture of DSPC, cholesterol, PEG2K-OMe and PEG3K-COOH at the ratio of 2:1:0.19:0.01 (m/m) was dissolved in chloroform and isopropyl ether. The resultant solution was added with a I-OHP solution (in a 9% sucrose solution) and then sonicated. The solution was evaporated at 60°C to remove the solvent and the lyophilization was repeated five times. The resultant product was sized at 60°C using EXTRUDER filter (twice at 400 nm and then five times at 100 nm), and then centrifuged twice at 200,000xg for 30 minutes. The precipitate was resuspended in a 9% sucrose solution or MES buffer (pH 5.5) to obtain I-OHP-PEG(-COOH/-OMe) liposomes.

Subsequently, PEG liposomes were derivatized with transferrin (Tf). The I-OHP-PEG(-COOH/-OMe) liposomes prepared as above were added with 1-ethyl-3-(3-dimethylamino-propyl)carbodiimide hydrochloride (EDC) (in an amount of 2.7% relative to the weight of the lipid components) and N-hydroxysulfosuccineimide (S-NHS) (in an amount of 7.3% relative to the weight of the lipid components), and the mixture was allowed to stand at room temperature for 10 minutes. The resultant solution was added with transferrin (Tf) (in an amount of 20% relative to the weight of the lipid components) and then stirred at room temperature for 3 hours. The solution was centrifuged at 200,000xg for 30 minutes, and the precipitate was resuspended in a-9% sucrose solution.

The apo-form of Tf(-PEG) liposomes prepared as above were added with iron citrate-sodium citrate and then stirred at room temperature for 15 minutes. The resultant solution was centrifuged at 200,000xg for 30 minutes. The precipitate was resuspended in a 9% sucrose solution to obtain holo-form of Tf(-PEG) liposomes.

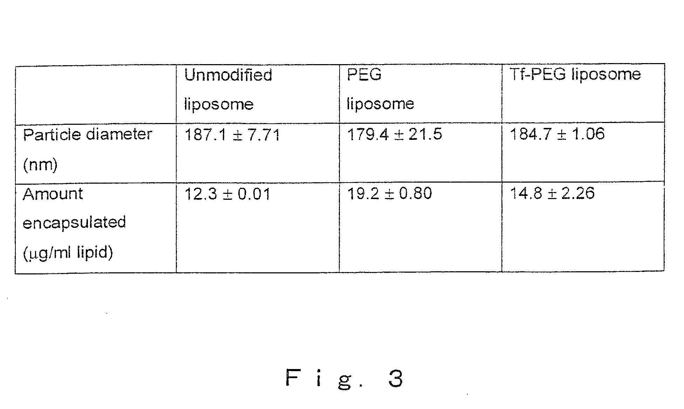

The characteristic properties of the unmodified liposome, PEG liposome and Tf-PEG liposome prepared as above are summarized in Fig. 3.

### EXAMPLE 3: Determination of the number of transferrin receptors on the cell surface

Human normal leukocytes and cells of different human malignant tumor-derived cell lines (K562, MKN45P and HL60) were used for the experiment. The number of TF receptors on the cell surface was determined by Scatchard analysis. A ¹²⁵I-labeled TF solution was added to a cell culture at different concentrations and incubated at 4°C for 1 hour. The concentration of TF was determined by protein quantification assay and, at the same time, the radioactivity was measured using a gamma counter. The solution was centrifuged to precipitate the cells, and the cell fraction was washed with an ice-cooled buffer and then measured with a gamma counter to determine the concentration of TF bound to the cell surface. The number of cells was determined by protein quantification assay. The concentration of unbound TF was determined by subtracting the concentration of bound TF from the known concentration of TF initially added. The number of bound TF (i.e., the number of the receptors) was determined from the Scatchard plot; the concentration of bound TF was plotted on the vertical axis and the ratio of the concentration of bound TF to the concentration of unbound TF was plotted on the horizontal axis, and the number of the bound TF (i.e., the number of the receptors) was determined from the x intercept of the graph.

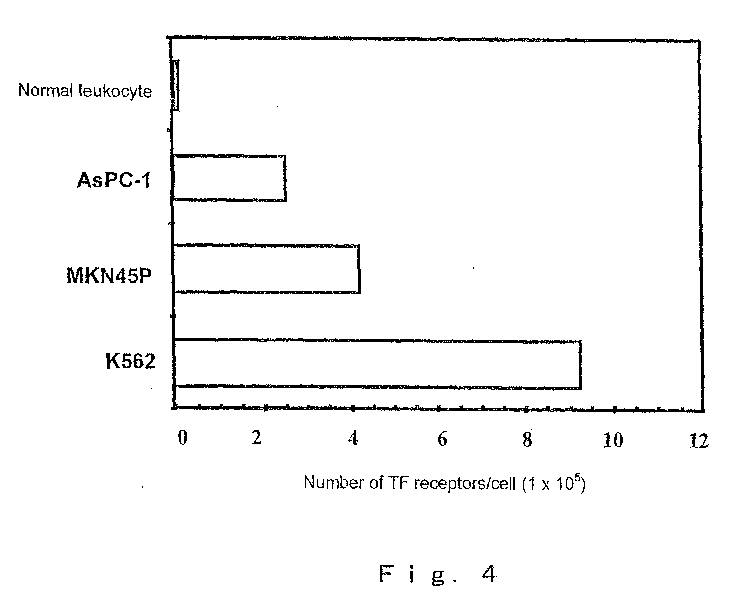

The number of ¹²⁵I-Tf bound to the cell surface in the different cell types are shown in Fig. 4. It was found that the number of transferrin receptors on the cell surface of the cell lines derived from the human malignant tumor was significantly higher than that in normal leukocytes.

### EXAMPLE 4: Therapeutic effect of I-OHP-containing liposome in peritonea inoculation model

Male BALB/c nu-nu nude mice aged 6 to 7 weeks were used as the animal models, and AsPC-1 cells (derived from human pancreatic cancer) and MKN45P cells (derived from human gastric cancer) were used as the tumor cells.

On day 0 of the experiment, AsPC-1 cells (2 x 10⁶ cells) or MKN45P cells(1 x 10⁷ cells) were intraperitoneally injected to the mice. On day 1 and day 4, the liposomes prepared in Example 2 or the I-OHP solution (8 mg/ml, in a 9% sucrose solution) was intraperitoneally injected to the mice. In either case, the concentration of oxaliplatin was adjusted to 5 mg I-OHP solution/kg body weight. As the liposomes to be administered, the Tf-PEG liposome, PEG liposome and unmodified liposome were used. As the negative control, PBS was administered.

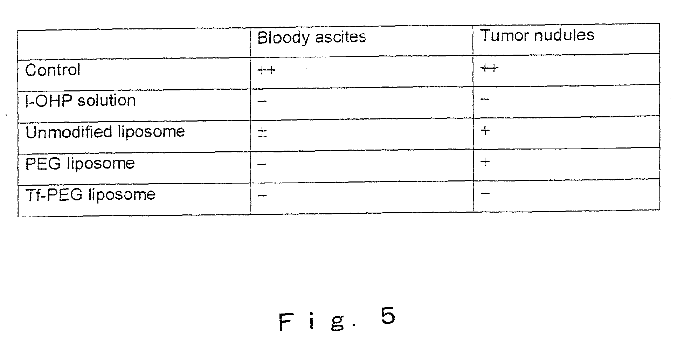

In the experiment, the mice were transabdominally incised on day 21 for the mice administered with AsPC-1 cells, and on day 16 and day 26 for the mice administered with MKN45P cells, to examine the presence or absence of bloody ascites and tumor nudules. The results are shown in Fig. 5.

It was found that the Tf-PEG liposome of the present invention could significantly reduce the appearance of bloody ascites and tumor nudules in the mice compared to those treated with unmodified liposomes or PEG liposomes.

### Industrial Applicability

The present invention provides a liposome preparation containing oxaliplatin and derivatized with a hydrophilic polymer. The liposome preparation of the invention is useful for treatment of cancers.

## Claims

1. A liposome preparation containing oxaliplatin and derivatized with a hydrophilic polymer and a ligand.

2. The liposome preparation according to Claim 1, wherein the ligand is selected from the group consisting of transferrin, folic acid, hyaluronic acid, a sugar chain, a monoclonal antibody and a Fab' fragment of a monoclonal antibody.

3. The liposome preparation according to Claim 1, wherein the ligand is transferrin.

4. The liposome preparation according to Claim 1, wherein the hydrophilic polymer is selected from the group consisting of polyethylene glycol, polymethylethylene glycol, polyhydroxypropylele glycol, polypropylene glycol, polymethylpropylene glycol and polyhydroxypropylene oxide.

5. The liposome preparation according to Claim 1, wherein the hydrophilic polymer is polyethylene glycol.

6. A pharmaceutical composition comprising a liposome preparation claimed in any one of Claims 1 to 5 and a pharmaceutically acceptable carrier.

7. Use of a liposome preparation as defined in any one of claims 1-5 for the preparation of a pharmaceutical composition for the treatment of a tumor.
